# EUROPEAN PATENT APPLICATION

(11) **EP 4 772 644 A1**
(43) Date of publication of application: **08.07.2026**
(21) Application number: 25762384.3
(22) Date of filing: 27.02.2025
(51) Int. Cl.: C12N 15/77, C12N 9/02, C12P 13/08, C12P 13/10, C12P 13/06

(54) **MICROORGANISM COMPRISING NICOTINAMIDE NUCLEOTIDE TRANSHYDROGENASE DERIVED FROM EDWARDSIELLA TARDA, AND METHOD FOR PRODUCING L-AMID ACIDS OR DERIVATIVES THEREOF USING SAME**

(30) Priority: 28.02.2024 KR 20240028603
(71) Applicant: CJ Cheiljedang Corporation, Seoul 04560 (KR)
(72) Inventor: KIM, Gyuree, Seoul 04560 (KR); JANG, Yongjae, Seoul 04560 (KR); HUH, Lan, Seoul 04560 (KR); KIM, Ju Eun, Seoul 04560 (KR); SHIN, Kwang Soo, Seoul 04560 (KR); KIM, Ye-Eun, Seoul 04560 (KR); KIM, Moonjung, Seoul 04560 (KR); KIM, Kyungrim, Seoul 04560 (KR); SHIM, Jihyun, Seoul 04560 (KR); KIM, Hyun Ah, Seoul 04560 (KR); JIN, Chorok, Seoul 04560 (KR); YOON, Jeong-Hye, Seoul 04560 (KR); EOM, Ga Eul, Seoul 04560 (KR); SEO, Chang Il, Seoul 04560 (KR); KIM, Seon Hye, Seoul 04560 (KR); YUN, Hyojin, Seoul 04560 (KR); KIM, Hye Mi, Seoul 04560 (KR)
(74) Representative: Meissner Bolte Partnerschaft mbB
(86) International application number: PCT/KR2025/002726
(87) International publication number: WO 2025/183467

(57) **Abstract**

The present disclosure relates to a microorganism of the genus *Corynebacterium* having improved ability to produce an L-amino acid or a derivative thereof, into which a heterologous nicotinamide nucleotide transhydrogenase protein is expressed or a polynucleotide encoding the same is introduced.

## Description

### [Technical Field]

The present disclosure relates to: a microorganism of the genus *Corynebacterium* having improved ability to produce an L-amino acid or a derivative thereof, into which a heterologous nicotinamide nucleotide transhydrogenase protein or a polynucleotide encoding the same is introduced; a method for producing an L-amino acid or a derivative thereof, comprising culturing the microorganism in a medium; a composition for producing an L-amino acid or a derivative thereof, comprising the microorganism, a culture of the microorganism, a fermentation product of the microorganism, or a combination of two or more thereof; and use of the microorganism for producing an L-amino acid or a derivative thereof.

### [Background Art]

Coryneform microorganisms are Gram-positive microorganisms that are frequently used for the industrial production of substances having various uses, such as feeds, pharmaceuticals, and foods, including L-amino acids and various nucleic acids. In recent years, diamines, keto-acids, and the like have also been produced from coryneform microorganisms.

In order to produce useful products through microbial fermentation, the demand for energy sources or reducing power increases along with enhancement of biosynthetic pathways for desired products within microorganisms. Among these, nicotinamide adenine dinucleotide phosphate (NADPH) is an essential factor for supplying reducing power. The oxidized form NADP⁺ and the reduced form NADPH function as electron carriers in the body and are involved in various biosynthetic processes. Among central metabolic pathways, pathways producing NADPH are known to mainly include: (1) the oxidative pentose phosphate pathway; and (2) production by an NADP-dependent isocitrate dehydrogenase (Icd gene) of the TCA pathway. In addition, various alternative pathways for supplying NADPH are present in many microorganisms, including a malate enzyme, glucose dehydrogenase, and non-phosphorylating glyceraldehyde-3-phosphate dehydrogenase.

Further, enzymes that produce NADPH independently of the central metabolic pathway include transhydrogenase and ferredoxin:NADP⁺ oxidoreductase.

As enhancement of various pathways and increased demand for energy sources or reducing power coexist, there remains a need for methods for increasing the ability to produce a desired L-amino acid or a derivative thereof.

### [Prior Art Documents]

### [Patent Documents]

(Patent Document 1) 1. U.S. Patent No. 7,629,142 B2

### [Disclosure]

### [Technical Problem]

The problem to be solved by the present disclosure is to provide a microorganism having improved ability to produce an L-amino acid or a derivative thereof, into which a heterologous nicotinamide nucleotide transhydrogenase is introduced.

### [Technical Solution]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below. Additionally, a number of papers and patent documents have been cited throughout the present specification. The content of the cited papers and patent documents is incorporated herein by reference in their entirety to describe the level of the technical field to which the present disclosure belongs and the contents of the present disclosure more clearly.

An aspect of the present disclosure provides a microorganism of the genus *Corynebacterium* having improved ability to produce an L-amino acid or a derivative thereof, into which a nicotinamide nucleotide transhydrogenase protein derived from *Edwardsiella tarda* or a polynucleotide encoding the same is introduced.

As used herein, the term "nicotinamide nucleotide transhydrogenase" refers to an enzyme that maintains cellular redox balance through the reaction [H⁺ + NADP⁺ + NADH ↔ H⁺ + NADPH + NAD⁺], and exists as a homodimer, each protomer including two soluble nucleotide-binding domains that mediate hydride transfer between NAD(H) and NADP(H) and a proton-translocating transmembrane domain. In the present disclosure, the term may refer to a transmembrane pyridine nucleotide transhydrogenase that catalyzes reduction of NADP⁺ to NADPH via oxidation of NADH to NAD⁺ and consists of two subunits encoded by the *pntA* and *pntB* genes, respectively. Specifically, the nicotinamide nucleotide transhydrogenase of the present disclosure is a protein encoded by a *pntAB* (*pntA* and *pntB*) gene and exhibits nicotinamide nucleotide transhydrogenase activity, and may be used interchangeably with PntAB. However, the type of the nicotinamide nucleotide transhydrogenase is not particularly limited, as long as it exhibits an activity corresponding to that of nicotinamide nucleotide transhydrogenase. The nicotinamide nucleotide transhydrogenase encoded by a *pntAB* gene is known in the art, and the amino acid and polynucleotide sequences of the nicotinamide nucleotide transhydrogenase can be obtained from a known database, examples of which include GenBank of NCBI, but are not limited thereto.

In one example, the nicotinamide nucleotide transhydrogenase derived from *Edwardsiella tarda* may consist of an alpha subunit PntA and a beta subunit PntB.

Additionally, the alpha subunit PntA of the nicotinamide nucleotide transhydrogenase protein derived from *Edwardsiella tarda* may consist of or comprise the amino acid sequence of SEQ ID NO: 18 or an amino acid sequence having at least 60% homology or identity thereto, and the beta subunit PntB may consist of or comprise the amino acid sequence of SEQ ID NO: 19 or an amino acid sequence having at least 60% sequence homology or identity thereto, but PntA and PntB are not limited thereto, as long as they exhibit nicotinamide nucleotide transhydrogenase protein activity. Specifically, it is apparent that even if a protein comprises an amino acid sequence in which part of the amino acid sequences of SEQ ID NOS: 18 and 19 is deleted, modified, substituted, or added, the protein may fall within the scope of the nicotinamide nucleotide transhydrogenase protein of the present disclosure as long as the protein exhibits efficacy corresponding to that of the nicotinamide nucleotide transhydrogenase protein. Additionally, PntA and PntB of the nicotinamide nucleotide transhydrogenase protein derived from *Edwardsiella tarda* may have or comprise the amino acid sequences having at least 60%, 70%, 80%, 85%, 90%, 95%, 96%, 97%, 98%, or 99% or more homology or identity to the amino acid sequences of SEQ ID NOS: 18 and 19, or consist of or essentially consist of the amino acid sequences. It is apparent that any amino acid sequence in which part of the sequence is deleted, modified, substituted, or added may also fall within the scope of the present disclosure, as long as the amino acid sequence has such a homology or identity and exhibits an equivalent efficacy to the protein.

Even if a polypeptide (or a protein) comprising an amino acid sequence set forth in a specific sequence number, a polypeptide (or a protein) consisting of an amino acid sequence set forth in a specific sequence number, or a polypeptide (or a protein) having an amino acid sequence set forth in a specific sequence number is disclosed in the present disclosure, it is apparent that a protein having an amino acid sequence in which part of the sequence has been deleted, modified, substituted, or added may also be used in the present disclosure, as long as it exhibits an identical or corresponding activity to that of the polypeptide or protein consisting of the amino acid sequence set forth in the corresponding sequence number. For example, this includes cases of having sequence addition that does not alter the function of the protein, naturally occurring mutation, silent mutation thereof, or conservative substitution at the N-terminus and/or C-terminus of the amino acid sequence.

The term "conservative substitution" refers to the substitution of an amino acid with a different amino acid having similar structural and/or chemical properties. Such an amino acid substitution may generally occur based on similarity in polarity, charge, solubility, hydrophobicity, hydrophilicity, and/or amphipathic nature of the residues. Typically, a conservative substitution may hardly affect or have no effect on the activity of the proteins.

As used herein, the term "identity" or "homology'" refers to the degree of similarity between two given amino acid sequences or nucleotide sequences and may be expressed as a percentage. In the present disclosure, the terms "homology" and "identity" may often be used interchangeably.

The sequence homology or identity of conserved polynucleotides or polypeptides (including proteins) is determined by a standard alignment algorithm, and a default gap penalty established by the used program may be used together. Substantially, homologous or identical sequences can typically hybridize with the whole sequence or part of a sequence, corresponding to at least about 50%, 60%, 70%, 80%, or 90% of the full length, under moderately or highly stringent conditions. It is apparent that hybridization also comprises hybridization with a polynucleotide comprising a general codon or a codon considering codon degeneracy in the polynucleotide.

Whether any two polynucleotide or polypeptide (including protein) sequences have homology, similarity, or identity may be determined by, for example, a known computer algorithm such as the "FASTA" program using default parameters as in Pearson et al. (1988) [Proc. Natl. Acad. Sci. USA 85]: 2444. Alternatively, it may be determined by the Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (the GCG program package (Devereux, J. et al., Nucleic Acids Research 12:387 (1984)) , BLASTP, BLASTN, and FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073) are included). For example, the homology, similarity, or identity may be determined by using BLAST of the National Center for Biotechnology Information or ClustalW.

The homology, similarity, or identity of polynucleotides or polypeptides (including proteins) may be determined by comparing sequence information using, for example, a GAP computer program, such as Needleman et al. (1970), J Mol Biol. 48:443, as disclosed in Smith and Waterman, Adv. Appl. Math (1981) 2:482. Briefly, the GAP program may be defined as the value obtained by dividing the number of similarly aligned symbols (*i.e.*, nucleotides or amino acids) by the total number of symbols in the shorter of the two sequences. The default parameters for the GAP program may include: (1) a binary comparison matrix (containing a value of 1 for identity and 0 for non-identity) and the weighted comparison matrix of Gribskov et al. (1986) Nucl. Acids Res. 14:6745 as disclosed in Schwartz and Dayhoff, eds., Atlas of Protein Sequence and Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps.

Further, whether any two polynucleotide sequences have homology, similarity, or identity may be identified by comparing the sequences in a Southern hybridization experiment under appropriate hybridization conditions. The appropriate hybridization conditions are within the corresponding technical range, and may be determined by a method well known to those skilled in the art (*e.g.*, J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York). However, the conditions are not limited thereto.

As used herein, the term "polynucleotide" refers to a DNA strand having at least a certain length, which is a polymer of nucleotides in which nucleotide monomers are connected in a long chain shape by covalent bonds.

Additionally, the sequence of a polynucleotide encoding the nicotinamide nucleotide transhydrogenase protein derived from *Edwardsiella tarda* having the amino acid sequence of SEQ ID NOS: 18 and 19 or an amino acid sequence having at least 60% homology or identity thereto can be obtained, for example, based on codon information known in the art. In one example, the alpha subunit PntA and beta subunit PntB of the nicotinamide nucleotide transhydrogenase protein may be encoded by a polynucleotide that may have or comprise the sequences of SEQ ID NOS: 28 and 29 or a base sequence having at least 60%, at least 70%, at least 75%, at least 80%, at least 85%, at least 90%, at least 95%, at least 96%, at least 97%, at least 98%, or at least 99% homology or identity to the sequences of SEQ ID NOS: 28 and 29, or consist of or essentially consist of the base sequences, but are not limited thereto.

Additionally, the base sequences of SEQ ID NOS: 28 and 29 can be obtained from a known database, examples of which include GenBank of NCBI, but are not limited thereto.

In the present disclosure, the polynucleotide (gene) comprising the base sequences of SEQ ID NOS: 28 and 29 may be used interchangeably with polynucleotide (gene) having the base sequences of SEQ ID NOS: 28 and 29, polynucleotide (gene) consisting of the base sequences of SEQ ID NOS: 28 and 29, or *pntAB.*

The polynucleotide of the present disclosure may undergo various modifications in the coding region within the range that does not change the amino acid sequence of the nicotinamide nucleotide transhydrogenase protein of the present disclosure, taking into consideration codon degeneracy or codons preferred in the organism in which the nicotinamide nucleotide transhydrogenase protein of the present disclosure is to be expressed. Accordingly, it is apparent that a polynucleotide that can be translated, by codon degeneracy, into a polypeptide consisting of an amino acid sequence of the nicotinamide nucleotide transhydrogenase protein of the present disclosure or a polypeptide having at least 60% homology or identity thereto may also be comprised in the polynucleotide of the present disclosure. For example, the polynucleotide of the present disclosure may be the sequences of SEQ ID NOS: 28 and 29 or a degenerate sequence thereof.

In another example, the polynucleotide of the present disclosure may have or comprise a base sequence having at least 60%, at least 70%, at least 75%, at least 80%, at least 85%, at least 90%, at least 95%, at least 96%, at least 97%, at least 98%, or at least 99% homology or identity to the sequence of SEQ ID NOS: 28 and 29, or may consist of or essentially consist of a base sequence having at least 60%, at least 70%, at least 75%, at least 80%, at least 85%, at least 90%, at least 95%, at least 96%, at least 97%, at least 98%, or at least 99% homology or identity to the sequence of SEQ ID NO: 2, but is not limited thereto.

Additionally, the polynucleotide of the present disclosure may hybridize with, a probe that can be prepared from a known gene sequence, for example, a sequence complementary to all or part of the polynucleotide sequence of the present disclosure under stringent conditions, and may comprise a sequence without limitation, as long as it is a sequence encoding the nicotinamide nucleotide transhydrogenase protein of the present disclosure.

As used herein, the term "stringent conditions" refers to conditions that enable specific hybridization between polynucleotides. These conditions are specifically described in the literature (such as J. Sambrook *et al.*, *supra*). For example, the stringent conditions may comprise conditions under which polynucleotides having high homology or identity, specifically polynucleotides having at least 40%, at least 50%, at least 60%, at least 70%, at least 75%, at least 80%, at least 85%, or at least 90%, more specifically polynucleotides having at least 95%, at least 96%, at least 97%, or at least 98%, or even more specifically polynucleotides having at least 99% homology or identity, hybridize with each other, while polynucleotides having homology or identity lower than the above do not hybridize with each other; or may comprise common washing conditions of Southern hybridization, *i.e.*, washing once, specifically twice or three times, at a salt concentration and temperature corresponding to 60°C, 1XSSC, 0.1% SDS, specifically 60°C, 0.1XSSC, 0.1% SDS, and more specifically 68°C, 0.1XSSC, 0.1% SDS.

Hybridization may occur between nucleotides having complementary base sequences, but the hybridized polynucleotides may include some base mismatches depending on the stringency of the hybridization. The term "complementary" is used to describe a relationship between nucleotide bases that can hybridize with each other. For example, regarding DNA, adenine is complementary to thymine, and cytosine is complementary to guanine. Therefore, the polynucleotide of the present disclosure may also include an isolated nucleic acid fragment complementary to the entire sequence as well as a nucleic acid sequence substantially similar thereto.

Specifically, polynucleotides having homology or identity may be detected using hybridization conditions comprising hybridization at a Tₘ value of 55°C, as well as the above-described conditions. In addition, the Tₘ value may be 60°C, 63°C, or 65°C, but is not limited thereto, and may be appropriately adjusted by those skilled in the art.

The appropriate stringency for hybridizing a polynucleotide depends on the length and degree of complementarity of the polynucleotide, and the variables are well known in the relevant technical field (see J. Sambrook *et al.*, *supra*).

For example, homologous or identical polynucleotide sequences can typically hybridize under stringent conditions along the whole sequence or at least about 50%, 60%, 70%, 80%, or 90% of the full length.
in the present disclosure, "vector" refers to a DNA construct for delivering a desired polynucleotide into a suitable host or host cell. For example, it may comprise a base sequence of a polynucleotide encoding a desired polypeptide or protein operably linked to a suitable expression regulatory region (or expression control sequence) such that the desired polypeptide or protein may be expressed in a suitable host, but is not limited thereto.

The expression regulatory region may comprise a promoter capable of initiating transcription, any operator sequence for regulating the transcription, a sequence encoding a suitable mRNA ribosome-binding site, and a sequence for regulating termination of transcription and translation. The vector, after being transformed into a suitable host cell (microorganism), may replicate or function independently of the host genome, or may be integrated into the genome itself to replicate or function.

The vector of the present disclosure may be an insertion vector for inserting a polynucleotide for introducing or increasing the activity of the heterologous nicotinamide nucleotide transhydrogenase protein of the present disclosure into a chromosome, but is not limited thereto. The insertion of a polynucleotide into a chromosome may be performed by any method known in the art, such as homologous recombination, but is not limited thereto. The vector may further comprise a selection marker to confirm transformation into a host cell, or further, insertion into the chromosome of the host cell. The selection marker is for selecting cells transformed with the vector, or for confirming the chromosomal insertion of a desired polynucleotide, and markers that confer selectable phenotypes such as drug resistance, auxotrophy, resistance to cytotoxic agents, or expression of surface polypeptides or proteins may be used. In an environment treated with a selective agent, only cells expressing the selection marker survive or exhibit other phenotypic traits, thereby enabling selection of transformed cells. The insertion vector may not comprise an origin of replication required for replication in transformed cells.

The vector used in the present disclosure is not particularly limited, and any vector known in the art may be used. Examples of commonly used vectors include plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, Charon21A, *etc.* may be used; and as a plasmid vector, those based on pDZ, pBR, pUC, pBluescriptII, pGEM, pTZ, pCL, pET, *etc.* may be used. Specifically, pDZ, pDC, pDCM2, pDC24, pACYC177, pACYC184, pCL, pECCG117, pUC19, pBR322, pMW118, and pCC1BAC vectors, *etc.* may be used.

As used herein, the term "transformation" refers to the introduction of a desired polynucleotide and/or a vector comprising the same into a host cell (microorganism), thereby altering the genetic traits of the host cell (microorganism). In the present disclosure, the transformation may mean introduction of a vector comprising a polynucleotide for introducing or increasing the activity of a heterologous nicotinamide nucleotide transhydrogenase protein into a host cell, thereby altering the genetic traits of the host cell. The transformed polynucleotide may be inserted into the chromosome of a host cell or located outside the chromosome. Additionally, the polynucleotide may comprise DNA and/or RNA encoding a desired protein (for example, a nicotinamide nucleotide transhydrogenase protein or *pntAB* protein). The polynucleotide may be introduced in a suitable form depending on the purpose of introduction. For example, a polynucleotide for the expression of a desired protein may be introduced into a host cell in the form of an expression cassette, which is a gene construct comprising all elements required for self-expression. The expression cassette may typically comprise a promoter operably linked to the coding sequence of the desired polynucleotide, a transcription termination signal, a ribosome binding site, and a translation termination signal. The expression cassette may be in the form of a self-replicating expression vector. Further, the polynucleotide may be introduced into a host cell as-isand be operably linked to a sequence required for expression in the host cell, but is not limited thereto.

As used herein, the term "operably linked" refers to a configuration in which a regulatory sequence is arranged at an appropriate position so as to control expression of a coding sequence. Hence, "operably linked" includes a regulatory region of a functional domain with a known or desired activity, such as a promoter, terminator, signal sequence, or enhancer region, being attached or linked to a target (gene or polypeptide) to regulate its expression, secretion, or function in accordance with the known or desired activity. For example, it may mean that a polynucleotide encoding a polypeptide is functionally linked to a promoter sequence that initiates and mediates transcription of the polynucleotide.

The method for transforming the vector of the present disclosure includes any method of introducing a nucleic acid into a cell and may be performed by selecting a suitable standard technique as known in the art depending on the host cell. For example, electroporation, calcium phosphate (CaPO₄) precipitation, calcium chloride (CaCl₂) precipitation, microinjection, a polyethylene glycol (PEG) method, a DEAE-dextran method, a cationic liposome method, and a lithium acetate-DMSO method may be used, but the method is not limited thereto.

As used herein, the term "microorganism (or strain)" includes both wild-type microorganisms and prokaryotic or eukaryotic microorganisms that have undergone genetic modification naturally or artificially. It may be a microorganism in which a specific mechanism is weakened or enhanced due to insertion of a foreign gene or enhancement or inactivation of activity of an endogenous gene, and may be a microorganism comprising a genetic modification for production of a desired polypeptide, protein, or product. In the present disclosure, the terms "microorganism", "strain", "host", and "host cell" may be used interchangeably.

As used herein, the term "recombinant microorganism" refers to a microorganism that has been genetically modified to exhibit a different genotype and/or phenotype compared to a naturally occurring microorganism (*e.g.*, when genetic modification affects how the nucleic acid sequence is encoded in the microorganism), and may comprise all progeny or potential progeny of the microorganism. In the present disclosure, the terms "recombinant microorganism", "genetically modified microorganism", "recombinant host cell", "recombinant cell", and "recombinant strain" may be used interchangeably. The recombinant microorganism may, for example, express a gene that is not found in its natural (non-recombinant) form, may not express a gene that is expressed in its natural form, or express a native gene in a manner different from that in which it is expressed in its natural form.

For the purpose of the present disclosure, the microorganism of the present disclosure may comprise any microorganism capable of producing a desired L-amino acid or a derivative thereof by introduction of a heterologous nicotinamide nucleotide transhydrogenase protein or a polynucleotide encoding the same. For example, the microorganism of the present disclosure may be characterized by having increased ability to produce an L-amino acid or a derivative thereof resulting from introduction of a heterologous nicotinamide nucleotide transhydrogenase protein or a polynucleotide encoding the same into the microorganism, and the microorganism may be a genetically engineered microorganism or a recombinant microorganism, but is not limited thereto. Specifically, the recombinant strain having increased ability to produce an L-amino acid or a derivative thereof may be a microorganism having increased ability to produce an L-amino acid or a derivative thereof compared to a natural wild-type microorganism, a non-modified microorganism having endogenous activity of nicotinamide nucleotide transhydrogenase, or a non-modified microorganism with no endogenous activity of nicotinamide nucleotide transhydrogenase, but is not limited thereto.

In one example, the microorganism having ability to produce an L-amino acid or a derivative thereof may be a prokaryotic or eukaryotic microorganism capable of producing an L-amino acid or a derivative thereof in a living organism, and comprises both a microorganism that inherently has the ability to produce an L-amino acid or a derivative thereof and a microorganism to which the ability to produce an L-amino acid or a derivative thereof has been imparted due to the activity of the heterologous nicotinamide nucleotide transhydrogenase protein of the present disclosure introduced into a parent strain lacking the ability to produce an L-amino acid or a derivative thereof. The ability to produce an L-amino acid or a derivative thereof may be imparted or enhanced by strain improvement.

The microorganism of the present disclosure may comprise any microorganism into which a heterologous nicotinamide nucleotide transhydrogenase protein or a polynucleotide encoding the same is introduced using various known methods.

In one example, the recombinant microorganism having improved ability to produce an L-amino acid or a derivative thereof of the present disclosure may comprise any microorganism having improved ability to produce an L-amino acid or a derivative thereof by introduction of a heterologous gene encoding the nicotinamide nucleotide transhydrogenase of the present disclosure, specifically nicotinamide nucleotide transhydrogenase derived from *Edwardsiella tarda*.

For example, the microorganism having improved ability to produce an L-amino acid or a derivative thereof may be a microorganism into which a polynucleotide encoding a protein consisting of the amino acid sequences of SEQ ID NOS: 18 and 19 or a protein comprising an amino acid sequence having at least 60%, 70%, 75%, 80%, 85%, 90%, 95%, 96%, 97%, 98%, 99%, 99.5%, 99.7%, or 99.9% homology or identity to the amino acid sequences of SEQ ID NOS: 18 and 19 is introduced.

For example, the microorganism having improved ability to produce an L-amino acid or a derivative thereof may be a microorganism into which a polynucleotide capable of encoding a protein comprising an amino acid sequence having at least 80% homology with the amino acid sequences of SEQ ID NOS: 18 and 19 or a polynucleotide comprising the base sequences of SEQ ID NOS: 28 and 29 or a base sequence having at least 60%, at least 70%, at least 75%, at least 80%, at least 85%, at least 90%, at least 95%, at least 96%, at least 97%, at least 98%, or at least 99% homology or identity to the base sequences of SEQ ID NOS: 28 and 29 is introduced.

In one example, the microorganism with improved ability to produce an L-amino acid or a derivative thereof of the present disclosure may be a microorganism with increased ability to produce an L-amino acid compared to a non-modified microorganism, but is not limited thereto. In one example, the non-modified microorganism, which is the reference strain for the comparison of increase in the ability to produce an L-amino acid , may be CA09-0903, CJ1R, KCCM12120P, KCCM11201P, or CJ3P strain, but is not limited thereto.

In one example, the recombinant microorganism having increased ability to produce an L-amino acid may exhibit an increase of at least about 1%, specifically, at least about 1%, at least about 2.5%, at least about 5%, at least about 6%, at least about 7%, at least about 8%, at least about 9%, at least about 10%, at least about 15%, at least about 16%, at least about 17%, at least about 18%, at least about 19%, at least about 20%, at least about 21%, at least about 22%, at least about 23%, at least about 24%, at least about 25%, at least about 26%, at least about 27%, at least about 28%, at least about 29%, at least about 30%, at least about 31%, at least about 32%, at least about 33%, at least about 34%, at least about 35%, at least about 36%, at least about 37%, at least about 39%, at least about 40%, at least about 45%, at least about 50%, at least about 55%, at least about 60%, at least about 65%, at least about 70%, at least about 75%, or at least about 80% (the upper limit is not particularly limited, for example, about 200% or less, about 150% or less, about 100% or less, about 90% or less, about 80% or less, about 70% or less, about 60% or less, about 50% or less, about 45% or less, about 40% or less, about 35% or less, about 30% or less, about 25% or less, or about 20% or less) compared to the ability to produce an L-amino acid of a parent strain before modification or a non-modified microorganism. However, the ability to produce an L-amino acid or a derivative thereof of the recombinant microorganism is not limited thereto, as long as it exhibits a positive increase compared to the ability of a parent microorganism (parent strain) before modification or a non-modified microorganism. In another example, the recombinant microorganism having increased ability to produce an L-amino acid may have an increase of at least about 1.1-fold, at least about 1.15-fold, at least about 1.16-fold, at least about 1.17-fold, at least about 1.18-fold, at least about 1.19-fold, at least about 1.2-fold, at least about 1.22-fold, at least about 1.23-fold, at least about 1.24-fold, at least about 1.25-fold, at least about 1.26-fold, at least about 1.27-fold, at least about 1.28-fold, at least about 1.29-fold, at least about 1.3-fold, at least about 1.31-fold, at least about 1.32-fold, at least about 1.33 fold, at least about 1.34-fold, at least about 1.35-fold, at least about 1.36-fold, at least about 1.37-fold, at least about 1.38-fold, at least about 1.39-fold, at least about 1.4-fold, at least about 1.45-fold, at least about 1.5-fold, at least about 1.55-fold, at least about 1.6-fold, at least about 1.65-fold, at least about 1.7-fold, at least about 1.75-fold, or at least about 1.8-fold (the upper limit is not particularly limited, for example, about 10-fold or less, about 5-fold or less, about 3-fold or less, about 2-fold or less, about 1.9-fold or less, about 1.8-fold or less, at most about 1.7-fold, at most about 1.6-fold, about 1.5-fold or less, at most about 1.4-fold, at most about 1.3-fold, or at most about 1.2-fold) compared to the ability to produce an L-amino acid of a parent microorganism (parent strain) before modification or a non-modified microorganism, but is not limited thereto.

The term "about" refers to a range encompassing ±0.5, ±0.4, ±0.3, ±0.2, ±0.1, *etc.*, including all numerical values in a range equal to or similar to the numerical value following the term "about", but the range is not limited thereto.

As used herein, the term "non-modified microorganism (strain)" does not exclude a microorganism (strain) comprising mutations that may occur naturally, and may refer to a wild-type microorganism (strain) or a natural microorganism (strain) as-is3, or a microorganism (strain) before a trait is altered by genetic variation due to natural or artificial factors. For example, the non-modified microorganism may mean a strain in which the nicotinamide nucleotide transhydrogenase protein of the present disclosure is not expressed, a strain in which the expression of the same is not increased, or a strain before the increase of the expression. Additionally, in one specific embodiment, the non-modified microorganism may mean a strain in which the heterologous nicotinamide nucleotide transhydrogenase protein of the present disclosure is not expressed, a strain in which the expression of the same is not increased, or a strain before the increase of the expression. In particular, the heterologous nicotinamide nucleotide transhydrogenase protein may be derived from *Edwardsiella tarda*, but is not limited thereto.

The "non-modified microorganism" may be used interchangeably with "microorganism (strain) before modification", "non-mutated microorganism (strain)", "parent strain", "parent microorganism", "wild-type microorganism (strain)", or "reference microorganism (strain)".

In one example, the microorganism having improved ability to produce an L-amino acid or a derivative thereof of the present disclosure may be either a prokaryotic cell or a eukaryotic cell, and specifically may be a prokaryotic cell. The prokaryotic cell may include, for example, microorganisms of the genus *Escherichia*, *Erwinia*, *Serratia*, *Providencia*, *Corynebacterium*, *Pseudomonas*, *Leptospira*, *Salmonella*, *Brevibacterium*, *Hyphomonas*, *Chromobacterium*, and *Nocardia*, or microorganisms belonging to fungi or yeast, but is not limited thereto. Specifically, it may be microorganisms of the genus *Escherichia*, *Corynebacterium*, and *Leptospira*, and yeast. More specifically, it may be a microorganism of the genus *Corynebacterium*.

As a microorganism according to any one of the foregoing specific embodiments, the microorganism may be a microorganism of the genus *Corynebacterium*.

In one embodiment of the present disclosure, the microorganism of the present disclosure may be *Corynebacterium glutamicum*, *Corynebacterium crudilactis*, *Corynebacterium deserti*, *Corynebacterium efficiens*, *Corynebacterium callunae*, *Corynebacterium stationis*, *Corynebacterium singulare*, *Corynebacterium halotolerans*, *Corynebacterium striatum*, *Corynebacterium ammoniagenes*, *Corynebacterium pollutisoli*, *Corynebacterium imitans*, *Corynebacterium testudinoris*, or *Corynebacterium flavescens*. Specifically, the microorganism of the present disclosure may be a microorganism of the genus *Corynebacterium*, and more specifically *Corynebacterium glutamicum*, but is not limited thereto.

Specifically, the microorganism of the present disclosure may be a microorganism of the genus *Corynebacterium*, and more specifically *Corynebacterium glutamicum*, but is not limited thereto.

Meanwhile, the microorganism of the genus *Corynebacterium* having improved ability to produce an L-amino acid of the present disclosure may comprise any natural wild-type microorganism, a microorganism of the genus *Corynebacterium* having improved ability to produce an L-amino acid or a derivative thereof by enhancing or weakening the expression of a gene related to the production mechanism of an L-amino acid, or a microorganism of the genus *Corynebacterium* having improved ability to produce an L-amino acid by introducing or enhancing an exogenous gene. In one specific embodiment, the microorganism may comprise a microorganism of the genus *Corynebacterium* having improved ability to produce an L-amino acid by increasing reducing power through the activity resulting from introduction of a heterologous nicotinamide nucleotide transhydrogenase protein into a non-modified microorganism, but is not limited thereto

As used herein, the term "increase" in protein (polypeptide) activity means that the activity of a protein (polypeptide) is increased in a host cell (microorganism) compared to its endogenous activity. The increase may be used interchangeably with terms such as "activation", "up-regulation", "overexpression", or "enhancement". The host cell (microorganism) may be a prokaryotic or eukaryotic microorganism.

The increase in protein (polypeptide) activity may comprise both the case in which a host cell (microorganism) exhibits a protein (polypeptide) activity that it did not inherently possess, and the case in which a host cell exhibits an improved protein (polypeptide) activity compared to its endogenous activity or its activity before modification.

For example, the case in which it "exhibits a protein (polypeptide) activity that it did not inherently possess" or the case in which it exhibits an improved protein (polypeptide) activity may result from "introduction of a protein (polypeptide)", but is not limited thereto.

As used herein, the term "introduction" of a protein (polypeptide) refers to a case in which a gene that a microorganism did not originally possess is expressed within the microorganism so that activity of a specific protein is exhibited, or to a case in which the polypeptide activity is enhanced, increased, or improved compared to its endogenous activity or its activity before modification. For example, it may result from the introduction of a gene encoding the protein (polypeptide) into a host cell (microorganism). For example, a polynucleotide encoding a specific protein (polypeptide) may be introduced into a chromosome of a host cell (microorganism), or a vector comprising the polynucleotide encoding a specific protein (polypeptide) may be introduced into a host cell (microorganism), thereby exhibiting or enhancing the activity thereof.

The "endogenous activity" refers to the activity of a specific protein (polypeptide) originally possessed by a host cell (microorganism) before transformation or a non-modified host cell (microorganism), when the trait is altered by genetic modification due to natural or artificial factors. This term may be used interchangeably with "activity before modification".

An increase in activity of a protein (polypeptide) compared to its endogenous activity means that the activity and/or concentration (expression level) of the protein (polypeptide) in a host cell (microorganism) is improved compared to the activity and/or concentration (expression level) of the protein (polypeptide) originally possessed by the host cell (microorganism) before modification or a non-modified host cell (microorganism).

In one example, the increase may refer to occurrence of the activity of the corresponding protein (polypeptide) that was absent, or increase in the activity or concentration of the protein, typically by at least about 1%, at least about 10%, at least about 25%, at least about 50%, at least about 75%, at least about 100%, at least about 150%, at least about 200%, at least about 300%, at least about 400%, or at least about 500%, up to at least about 1000% or at least about 2000% relative to the activity or concentration in a host cell (microorganism) before transformation or in a non-modified host cell (microorganism), but is not limited thereto.

The increase in activity of a protein (polypeptide) may be achieved by introducing a foreign protein (polypeptide) or by increasing the activity of an endogenous protein (polypeptide). Whether the activity of a protein (polypeptide) has increased may be confirmed from the increase in the activity level of the protein (polypeptide), its expression level, or the amount of product resulting from the activity of the corresponding protein (polypeptide).

The increase in the activity of a protein (polypeptide) can be achieved by various methods well known in the art, and is not limited as long as the activity of the desired protein (polypeptide) can be increased compared to that of the host cell (microorganism) before modification. Specifically, it may be achieved by using genetic engineering and/or protein engineering well known to those skilled in the art, which are routine molecular biology techniques, but is not limited thereto (*e.g.*, Sitnicka et al. Functional Analysis of Genes. Advances in Cell Biology. 2010, Vol. 2. 1-16, Sambrook et al. Molecular Cloning 2012, *etc.*).

Specifically, the increase in the activity of a protein (polypeptide) of the present disclosure may result from:
1) increase in the intracellular copy number of a polynucleotide encoding the protein (polypeptide);
2) modification of a gene expression regulatory region on a chromosome encoding the protein (polypeptide) (*e.g.*, introduction of a modification on the expression regulatory region, replacement with a sequence exhibiting stronger activity, or insertion of a sequence exhibiting stronger activity);
3) modification of a base sequence encoding a start codon or the 5'-UTR of a gene transcript encoding the protein (polypeptide);
4) modification of the amino acid sequence of the protein (polypeptide) to increase the activity of the protein (polypeptide);
5) modification of a polynucleotide sequence encoding the protein (polypeptide) to increase the activity of the protein (polypeptide) (*e.g.*, modification of a polynucleotide sequence of a gene encoding the protein (polypeptide), such that the gene encodes a protein (polypeptide) modified to have increased activity);
6) introduction of a foreign protein (polypeptide) exhibiting the activity of the protein (polypeptide), or a foreign polynucleotide encoding the same;
7) codon optimization of a polynucleotide encoding the protein (polypeptide);
8) selection and modification or chemical modification of exposed regions of the protein (polypeptide) through analysis of the tertiary structure thereof;
9) regulation of the cellular localization of the protein (polypeptide); or
10) a combination of two or more selected from 1) to 9) above, but is not particularly limited thereto.

For example,
The 1) increase in the intracellular copy number of a polynucleotide encoding the protein (polypeptide) may be achieved by introducing a vector comprising the polynucleotide encoding the protein (polypeptide) operably linked to a suitable regulatory sequence into a host cell (microorganism). Alternatively, it may be achieved by introducing one copy, or two or more copies, of a polynucleotide encoding the protein (polypeptide) operably linked to a suitable regulatory sequence into a chromosome of a host cell (microorganism). The introduction into a chromosome may be performed by introducing a vector capable of inserting the polynucleotide into a chromosome of a host cell (microorganism) into the host cell (microorganism), but is not limited thereto. The vector is as described above. With respect to the polynucleotide sequence encoding the protein, the regulatory sequence may be a native sequence (of the same origin) or a foreign sequence (derived from a different gene), a variant thereof, or another artificial sequence, and may induce expression of the polynucleotide in a host cell (microorganism). In one embodiment, the regulatory sequence of the gene (*pntAB*) encoding a nicotinamide nucleotide transhydrogenase protein derived from *Edwardsiella tarda* of the present disclosure may be replaced with a PgapA promoter, but is not limited thereto.

The 2) replacement of the expression regulatory region (or expression regulatory sequence) of a gene encoding the protein (polypeptide) on the chromosome with a sequence having strong activity may be achieved, for example, by introducing a modification into the sequence through deletion, insertion, substitution, or a combination thereof to further increase the activity of the expression regulatory region, or by replacing the sequence with a sequence having stronger activity. The expression regulatory region may comprise, but is not particularly limited to, a promoter, an operator sequence, a sequence encoding a ribosome-binding site, a sequence regulating the termination of transcription and translation, and the like. In one example, it may specifically comprise replacing the original promoter with a strong promoter, but is not limited thereto.

Examples of known strong promoters comprise cj1 to cj7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, and the like, but the strong promoter is not limited thereto.

The 3) modification of a base sequence encoding a start codon or the 5'-UTR of a gene transcript encoding the protein (polypeptide) may be achieved, for example, by modifying the base sequence such that it encodes a different initiation codon with a higher expression rate of the protein (polypeptide) compared to the endogenous initiation codon, or by modifying the base sequence such that it encodes a ribosome binding site (RBS) sequence with a higher expression rate of the protein (polypeptide) compared to the endogenous RBS sequence, but is not limited thereto.

The 4) and 5) modification of the amino acid sequence or polynucleotide sequence of the protein (polypeptide) may be achieved by introducing a modification on the sequence of the amino acid sequence of the protein (polypeptide) or the polynucleotide sequence encoding the protein (polypeptide) through deletion, insertion, substitution, or a combination thereof to enhance the activity of the protein (polypeptide), or by replacing the sequence with an amino acid sequence or polynucleotide sequence modified to have increased activity, but is not limited thereto. The replacement may specifically be performed by inserting the polynucleotide into a chromosome by homologous recombination, but is not limited thereto.

The 6) introduction of a foreign polynucleotide exhibiting the activity of the protein (polypeptide) may be achieved by introducing into a host cell (microorganism) a foreign polynucleotide encoding a protein (polypeptide) that exhibits the same/similar activity to that of the protein (polypeptide). The foreign polynucleotide is not limited by its origin or sequence, as long as it exhibits the same/similar activity to that of the protein (polypeptide). The introduction may be performed by a transformation method known in the art appropriately selected by those skilled in the art, and the expression of the introduced polynucleotide in the host cell enables the production of the protein (polypeptide), thereby increasing its activity.

The 7) codon optimization of a polynucleotide encoding the protein (polypeptide) may be achieved by codon-optimizing an endogenous polynucleotide to increase transcription or translation within a host cell (microorganism), or by optimizing the codons of a foreign polynucleotide such that optimized transcription and translation thereof may be achieved within the host cell.

The 8) selection and modification or chemical modification of exposed regions of the protein (polypeptide) through analysis of the tertiary structure thereof may be achieved, for example, by comparing the sequence information of the protein (polypeptide) to be analyzed with a database storing the sequence information of known proteins to identify template protein candidates according to the degree of sequence similarity and confirming the structure based on the information, thereby selecting the exposed site to be modified or chemically modified and transforming or modifying the same.

The 9) regulation of the cellular localization of the protein (polypeptide) may be achieved by targeting the protein (polypeptide) to a particular intracellular organelle or a particular intracellular space. For example, it may be achieved by targeting the protein (polypeptide) to the periplasm or cytoplasm through the addition or removal of a leader sequence that functions in the targeting of proteins (polypeptides), but is not limited thereto.

Such enhancement of the protein (polypeptide) activity may refer to an increase in the activity or concentration of the corresponding protein (polypeptide) relative to the activity or concentration of the protein (polypeptide) expressed in a wild-type strain or a host cell (microorganism) before modification, or an increase in the amount of product produced by the protein (polypeptide), but is not limited thereto.

The modification of a part or the entirety of a polynucleotide in the microorganism of the present disclosure may be induced by: (a) a method using homologous recombination using a vector for chromosomal insertion into the microorganism or genome editing using an engineered nuclease (*e.g.*, CRISPR-Cas9), and/or (b) treatment with light, such as ultraviolet light and radiation, and/or chemicals, but is not limited thereto.

The microorganism of the present disclosure may have improved ability to produce an L-amino acid or a derivative thereof.

In the present disclosure, "L-amino acid or a derivative thereof" comprises any L-amino acid or a derivative thereof that can be produced by a microorganism through metabolic processes from various carbon sources, and specifically may comprise L-lysine, L-valine, L-arginine, L-threonine, and L-homoserine, but is not limited thereto.

In one specific embodiment of the present disclosure, the microorganism of the genus *Corynebacterium* may have increased ability to produce an L-amino acid or a derivative thereof compared to a non-modified microorganism, wherein the L-amino acid is at least one selected from L-lysine, L-valine, L-arginine, L-threonine, and L-homoserine.

Still another aspect of the present disclosure provides a method for producing an L-amino acid or a derivative thereof, comprising culturing, in a medium, a microorganism of the genus *Corynebacterium* having improved ability to produce an L- amino acid or a derivative thereof, into which a nicotinamide nucleotide transhydrogenase protein derived from *Edwardsiella tarda* of the present disclosure or a polynucleotide encoding the same is introduced.

The microorganism is as described in other aspects above.

As used herein, the term "culturing" refers to growing the microorganism of the present disclosure under properly controlled environmental conditions. Herein, the culturing process may be performed in a suitable medium known in the art under suitable culturing conditions known in the art. Such a culturing process may be easily adjusted and used by those skilled in the art according to the selected strain. Specifically, the culturing may be batch culturing, continuous culturing, and fed-batch culturing, but is not limited thereto.

The microorganism of the present disclosure may be cultured in a common medium containing appropriate carbon sources, nitrogen sources, phosphorus sources, inorganic compounds, amino acids and/or vitamins, *etc.*, while controlling the temperature, pH, *etc.* under aerobic conditions.

In the present disclosure, the carbon sources include carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamic acid, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (*i.e.*, molasses converted into reducing sugars) may be used, and other various carbon sources in appropriate amounts may be used without limitation. These carbon sources may be used alone or in combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamic acid, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of two or more, but are not limited thereto.

As the phosphorus sources, monobasic potassium phosphate, dibasic potassium phosphate, or the corresponding sodium-containing salts may be used. As for inorganic compounds, sodium chloride, calcium chloride, iron chloride, magnesium sulfate, iron sulfate, manganese sulfate, calcium carbonate, *etc.*, may be used, and in addition, amino acids, vitamins, and/or suitable precursors may be comprised. These components or precursors may be added to the medium in a batchwise or continuous manner. However, the medium is not limited thereto.

During the culturing of the microorganism of the present disclosure, the pH of the medium may be adjusted by adding compounds such as ammonium hydroxide, potassium hydroxide, ammonia, phosphoric acid, sulfuric acid, *etc.* to the medium in a suitable manner. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas or nitrogen, hydrogen, or carbon dioxide gas may be injected. However, the culturing conditions are not limited thereto.

Further, the culture medium may contain metal salts such as magnesium sulfate or iron sulfate necessary for growth. Lastly, in addition to the aforementioned substances, essential growth substances such as amino acids and vitamins may be used. Additionally, suitable precursors may be used in the culture medium. The aforementioned raw materials may be added to the culture in a batch or continuous manner by a suitable method during the culturing process, but are not limited thereto.

During the culturing of the microorganism of the present disclosure, the pH of the culture may be adjusted by adding compounds such as ammonium hydroxide, potassium hydroxide, ammonia, phosphoric acid, sulfuric acid, *etc.* to the culture in a suitable manner. During the culturing, an antifoaming agent such as fatty acid polyglycol ester may be used to suppress foaming. Additionally, oxygen or oxygen-containing gas may be injected into the culture in order to maintain the aerobic state of the culture, or gas may not be injected or nitrogen, hydrogen, or carbon dioxide gas may be injected in order to maintain the anaerobic and microaerobic states, but are not limited thereto.

In the culturing of the present disclosure, the culturing temperature may be maintained at 20°C to 35°C, specifically 25°C to 35°C, and the culturing period may be continued until a yield of a useful substance is obtained, and may be about 10 to 160 hours, about 20 to 130 hours, about 24 to 120 hours, about 36 to 120 hours, about 48 to 120 hours, about 48 hours or more, or about 48 hours, about 72 hours, or about 120 hours, but is not limited thereto.

An L-amino acid or a derivative thereof produced by the culturing of the present disclosure may be secreted into the medium or remain in cells.

In one specific embodiment of the present disclosure, the L-amino acid or a derivative thereof may be at least one selected from L-lysine, L-valine, L-arginine, L-threonine, and L-homoserine, but is not limited thereto.

In one specific embodiment, the method for producing an L-amino acid or a derivative thereof of the present disclosure may further comprise preparing the microorganism of the present disclosure, preparing a medium for culturing the microorganism, or a combination thereof (in any order), for example, prior to culturing.

The method for producing an L-amino acid or a derivative thereof of the present disclosure may further comprise recovering a desired substance, specifically the L-amino acid or a derivative thereof, from the cultured microorganism, the culture of the microorganism, the fermentation product of the microorganism, or the culture medium. The recovering step may be further included after the culturing.

The recovery may be collecting the desired L-amino acid or a derivative thereof using a suitable method known in the art according to the method for culturing a microorganism of the present disclosure, for example, batch, continuous, or fed-batch culturing. For example, the recovery may involve centrifugation, filtration, treatment with a crystallized protein precipitating agent (salting-out method), extraction, sonication, ultrafiltration, dialysis, various kinds of chromatography such as molecular sieve chromatography (gel filtration), adsorption chromatography, ion exchange chromatography, and affinity chromatography, HPLC or a combination thereof. A desired substance, specifically an L-amino acid or a derivative thereof, can be recovered from the medium or microorganism using a suitable method known in the art.

Additionally, the method for producing an L-amino acid or a derivative thereof of the present disclosure may further comprise purification. The purification may be performed using a suitable method known in the art. In one example, when the method for producing an L-amino acid or a derivative thereof of the present disclosure comprises both recovery and purification, the recovery and purification may be performed continuously or discontinuously in any order, simultaneously, or as one integrated step, but are not limited thereto.

In the method of the present disclosure, the nicotinamide nucleotide transhydrogenase derived from *Edwardsiella tarda*, introduction, and L-amino acid or a derivative thereof are as described in other aspects above.

Still another aspect of the present disclosure provides a composition for producing an L-amino acid or a derivative thereof, comprising: a microorganism of the genus *Corynebacterium* having improved ability to produce an L-amino acid or a derivative thereof, into which a polynucleotide encoding a nicotinamide nucleotide transhydrogenase protein derived from *Edwardsiella tarda* is introduced; a culture of the microorganism; a fermentation product of the microorganism; or a combination of two or more thereof.

The composition of the present disclosure may further comprise any appropriate excipient that is commonly used in compositions for producing an L-amino acid or a derivative thereof. Such excipient may be, for example, a preservative, a wetting agent, a dispersing agent, a suspending agent, a buffering agent, a stabilizing agent, an isotonic agent, *etc.*, but is not limited thereto.

In one specific embodiment, the composition of the present disclosure may comprise each component in a microbially effective amount or in an amount that can be suitably present in a composition for production.

In the composition of the present disclosure, the nicotinamide nucleotide transhydrogenase derived from *Edwardsiella tarda*, introduction, and L-amino acid or a derivative thereof are as described in other aspects above.

Still another aspect of the present disclosure provides a use of a microorganism of the genus *Corynebacterium* having improved ability to produce an L-amino acid or a derivative thereof, into which a nicotinamide nucleotide transhydrogenase protein derived from *Edwardsiella tarda* or a polynucleotide encoding the same is introduced, for producing the L-amino acid or a derivative thereof.

In the use of the present disclosure, the nicotinamide nucleotide transhydrogenase derived from *Edwardsiella tarda*, introduction, and L-amino acid or a derivative thereof are as described in other aspects above.

Still another aspect of the present disclosure provides a method for producing a microorganism of the genus *Corynebacterium* having improved ability to produce an L-amino acid or a derivative thereof, comprising introducing a nicotinamide nucleotide transhydrogenase protein derived from *Edwardsiella tarda* or a polynucleotide encoding the same into a microorganism of the genus *Corynebacterium* having ability to produce an L-amino acid or a derivative thereof.

Still another aspect of the present disclosure provides a method for increasing ability to produce an L-amino acid or a derivative thereof, comprising introducing a nicotinamide nucleotide transhydrogenase protein derived from *Edwardsiella tarda* or a polynucleotide encoding the same into a microorganism of the genus *Corynebacterium* having ability to produce an L-amino acid or a derivative thereof.

In the method of the present disclosure, the nicotinamide nucleotide transhydrogenase derived from *Edwardsiella tarda*, introduction, and L-amino acid or a derivative thereof are as described in other aspects above.

### [Mode for Carrying Out the Invention]

Hereinafter, the present disclosure will be described in more detail by way of Examples and Experimental Examples. However, these Examples and Experimental Examples are given for illustrative purposes only, and the scope of the present disclosure is not intended to be limited by these Examples and Experimental Examples.

### Example 1: Construction of recombinant vectors for introduction of a heterologous nicotinamide nucleotide transhydrogenase (pntAB)

### Example 1-1: Construction of plasmids for gene insertion

In order to insert a heterologous nicotinamide nucleotide transhydrogenase (hereinafter, *pntAB*) gene into the chromosome of *Corynebacterium glutamicum*, NCgl1287, which is known as a gene encoding a transposon, was used as an insertion site (J. Bacteriol. 2011 Mar; 193(5): 1237-1249). To replace the NCgl1287 gene with a heterologous *pntAB* gene, an NCgl1287-deletion and target gene insertion vector was constructed. To construct the vector, PCR was performed using the chromosomes of ATCC 13032, ATCC 13869, and ATCC 14067 as templates with primer pairs of SEQ ID NOS: 1 and 2, and SEQ ID NOS: 3 and 4. The primer sequences used for each PCR are shown in Table 1 below.

For the PCR reaction, PfuUltra^{™} high-fidelity DNA polymerase (Stratagene) was used as a polymerase, and the PCR was performed under conditions of denaturation at 95°C for 30 seconds, annealing at 55°C for 30 seconds, and extension at 72°C for 1 minute, and the denaturation, annealing, and extension were repeated for 28 cycles. As a result, each obtained DNA product was purified using a PCR purification kit (PCR Purification Kit, QIAGEN). The purified amplification products were treated with the restriction enzyme SmaI, and then cloned, together with a pDC24 vector (SEQ ID NO: 38) that had been heat-treated at 65°C for 20 minutes, using an In-Fusion Cloning Kit (TaKaRa) according to the provided manual, thereby constructing NCgl1287-deletion and target gene insertion vectors pDC24ΔN1287(13032), pDC24ΔN1287(13869), and pDC24ΔN1287(14067).

**[Table 1]**

| SEQ ID NO | Name | Sequence |
|---|---|---|
| 1 | Primer | AATTCGAGCTCGGTACCCAATGGAGCTGAAAGAAT |
| 2 | Primer | CTTCCTGATAGTCCCGGGACATTGTTTTTC |
| 3 | Primer | GAAAAACAATGTCCCGGGACTATCAGGAAG |
| 4 | Primer | GTCGACTCTAGAGGATCCCCATAAAAACAGGCAGAGGA |

### Example 1-2: Construction of plasmids for promoter replacement

In order to further enhance the expression of the *pntAB* gene, an expression cassette was constructed in which the endogenous promoter of the *pntAB* gene was replaced with a promoter of the *gapA* gene (PgapA).

Specifically, in order to construct a strain into which the heterologous *pntAB* gene regulated by the PgapA promoter was introduced, a PgapA promoter fragment was obtained by using chromosomal DNA of *Corynebacterium glutamicum* 13032 as a template and SEQ ID NOS: 5 and 6.

Additionally, information on the amino acid sequences and base sequences of genes encoding *pntAB* derived from *Escherichia coli*, *Corynebacterium urealyticum*, *Corynebacterium amycolatum*, and *Cellulosimicrobium funkei* was obtained from the National Institutes of Health GenBank (NIH GenBank), and codon-optimized *pntAB* genes derived from each microorganism were synthesized using a gene synthesis service of Cosmo Genetech.

| Microorganism | pntA amino acid sequence | pntB amino acid sequence |
|---|---|---|
| Escherichia coli | WP_001300486. | WP_000014036.1 |
| *Corynebacterium* urealyticum | WP_012360758.1 | WP_012360759.1 |
| *Corynebacterium* Amycolatum | WP_149421085.1 | WP_149421086.1 |
| Cellulosimicrobium funkei | WP_047231978.1 | WP_047231810.1 |

PCR was performed using each of the synthesized heterologous *pntAB* genes and chromosomal DNA of *Edwardsiella tarda* (accession No. KACC 15172) as templates with primer pairs of SEQ ID NOS: 7 and 8; SEQ ID NOS: 9 and 10; SEQ ID NOS: 11 and 12; SEQ ID NOS: 13 and 14; and SEQ ID NOS: 15 and 16, respectively. The primer sequences used for each PCR are shown in Table 2 below.

**[Table 2]**

| SEQ ID NO | Name | Sequence |
|---|---|---|
| 5 | Primer | CCTGAAAAACAATGTCCCAACGACCGAGCCTATTG |
| 6 | Primer | GTTGTGTCTCCTCTAAAGAT |
| 7 | Primer | CTTTAGAGGAGACACAACATGCGTATTGGTGTAC |
| 8 | Primer | CCAACTTCCTGATAGTCCCTTATAATGCGCGTAG |
| 9 | Primer | AGAGGAGACACAACATGCGAATTGGCATACCA |
| 10 | Primer | AACTTCCTGATAGTCCCTTACAGAGCTTTCAGGAT |
| 11 | Primer | TCTTTAGAGGAGACACAACGTGCGTATCGGTATTC |
| 12 | Primer | ACCAACTTCCTGATAGTCCCTTAGTTGCTGATCCCTG C |
| 13 | Primer | ATCTTTAGAGGAGACACAACATGAGAATCGGCATCCC TT |
| 14 | Primer | CCAACTTCCTGATAGTCCCTTAAGCGTTGACCTT |
| 15 | Primer | ATCTTTAGAGGAGACACAACATGCGCATCGGTGTGCC |
| 16 | Primer | ACTTCCTGATAGTCCCTCAACCCACCCGCAGC |

For the PCR reaction, PfuUltra^{™} high-fidelity DNA polymerase (Stratagene) was used as a polymerase, and the PCR was performed under amplification conditions of denaturation at 95°C for 30 seconds, annealing at 55°C for 30 seconds, and extension at 72°C for 1 minute, and the denaturation, annealing, and extension were repeated for 28 cycles.

As a result, a 472 bp DNA fragment of the PgapA promoter region of SEQ ID NO: 17 was obtained, and a 2919 bp DNA fragment of the *pntAB* region of *Edwardsiella tarda*, a 2932 bp DNA fragment of the *pntAB* region of *Escherichia coli*, a 3045 bp DNA fragment of the *pntAB* region of *Corynebacterium urealyticum*, a 3066 bp DNA fragment of the *pntAB* region of *Corynebacterium amycolatum*, and a 3021 bp DNA fragment of the *pntAB* region of *Cellulosimicrobium funkei*, encoding the amino acid sequences set forth in SEQ ID NOS: 18 and 19; SEQ ID NOS: 22 and 23; SEQ ID NOS: 20 and 21; SEQ ID NOS: 22 and 23; SEQ ID NOS: 24 and 25; and SEQ ID NOS: 26 and 27, respectively, were obtained.

PCR was performed using the amplified promoter and the 2919 bp DNA fragment of the *pntAB* region of *Edwardsiella tarda* as templates and primers of SEQ ID NOS: 5 and 8; the amplified promoter and the 2932 bp DNA fragment of the *pntAB* region of *Escherichia coli* as templates and primers of SEQ ID NOS: 5 and 10; the amplified promoter and the 3045 bp DNA fragment of the *pntAB* region of *Corynebacterium urealyticum* as templates and primers of SEQ ID NOS: 5 and 12; the amplified promoter and the 3066 bp DNA fragment of the *pntAB* region of *Corynebacterium amycolatum* as templates and primers of SEQ ID NOS: 5 and 14; and the amplified promoter and the 3021 bp DNA fragment of the *pntAB* region of *Cellulosimicrobium funkei* as templates and primers of SEQ ID NOS: 5 and 16, respectively. The PCR was performed under PCR amplification conditions of denaturation at 95°C for 5 minutes, followed by 28 cycles of denaturation at 95°C for 30 seconds, annealing at 55°C for 30 seconds, and polymerization at 72°C for 2 minutes, and then polymerization at 72°C for 5 minutes.

As a result, a 3.4 kb heterologous *pntAB* DNA fragment encoding a heterologous nicotinamide nucleotide transhydrogenase linked to the PgapA promoter was amplified. The amplified product was purified using a PCR purification kit (PCR Purification Kit, QIAGEN) and used as an insert DNA fragment for vector construction.

The purified amplification product was treated with the restriction enzyme SmaI, and then cloned, at a molar concentration (M) ratio of 1:2 with pDC24△N1287(13032) or pDC24△N1287(13869) or pDC24△N1287(14067) vector that had been heat-treated at 65°C for 20 minutes, using an In-Fusion Cloning Kit (TaKaRa) according to the provided manual, thereby constructing vectors for introducing heterologous *pntAB* into the chromosome, namely pDC24△N1287(13032)::PgapA_pntAB(E.ta), pDC24△N1287(13869)::PgapA_pntAB(E.ta), pDC24△N1287(14067)::PgapA_pntAB(E.ta), pDC24△N1287(13032)::PgapA_pntAB(E.co), pDC24△N1287(13032)::PgapA_pntAB(C.ur), pDC24△N1287(13032)::PgapA_pntAB(C.am), and pDC24△N1287(13032)::PgapA_pntAB(C.fu).

### Example 2: Construction of strains producing an L-amino acid or a derivative thereof, into which heterologous pntAB was introduced, and evaluation of ability to produce an L-amino acid or a derivative thereof

### Example 2-1: Construction of L-threonine-producing strains into which heterologous pntAB was introduced and evaluation of threonine-producing ability

The five types of vectors constructed in Example 1 were transformed into an L-threonine-producing strain, *Corynebacterium glutamicum* KCCM 12502P (CA09-0903, Korean Patent No. 10-2126951), by electroporation to construct strains expressing heterologous *pntAB*.

In order to examine threonine productivity of the strains, culturing was performed as follows.

First, each strain was inoculated into a 250 mL corner-baffled flask containing 25 mL of seed medium, and incubated with shaking at 30°C for 20 hours at 200 rpm. Thereafter, 1 mL of the seed culture was inoculated into a 250 mL corner-baffled flask containing 24 mL of production medium, and incubated with shaking at 32°C for 24 hours at 200 rpm.

### [Seed Medium (pH 7.0)]

Glucose 20 g, Peptone 10 g, Yeast Extract 5 g, Urea 1.5 g, KH₂PO₄ 4 g, K₂HPO₄ 8 g, MgSO₄·7H₂O 0.5 g, Biotin 100 µg, Thiamine HCl 1000 µg, Calcium Pantothenate 2000 µg, and Nicotinamide 2000 µg (per 1 L of distilled water).

### [Production Medium (pH 7.0)]

Glucose 20 g, KH₂PO₄ 2 g, Urea 1.5 g, (NH₄)₂SO₄ 40 g, Peptone 2.5 g, CSL(Sigma) 5 g(10 mL), MgSO₄·7H₂O 0.5 g, Leucine 400 mg, and CaCO₃ 20 g (per 1 L of distilled water).

After completion of culturing, the amount of L-threonine produced was measured using high-performance liquid chromatography (HPLC), and the concentrations are shown in Table 3 below.

**[Table 3]**

| Threonine-producing ability of microorganism expressing *pntAB* derived from various microorganisms | | |
|---|---|---|
| Strain Name | L-threonine concentration (g/L) | Concentration relative to parent strain (%) |
| CA09-0903 (parent strain) | 3.4 | 100 |
| CA09-0903 | 5.9 | 174 |
| △N1287(13032)::PgapA_pntAB(E.ta) | | |
| CA09-0903 | 4.1 | 121 |
| △N1287(13032)::PgapA_pntAB(E.co) | | |
| CA09-0903 | 2.9 | 85 |
| △N1287(13032)::PgapA_pntAB(C.ur) | | |
| CA09-0903 | 3.5 | 103 |
| △N1287(13032)::PgapA_pntAB(C.am) | | |
| CA09-0903 | 1.7 | 50 |
| △N1287(13032)::PgapA_pntAB(C.fu) | | |

As a result, as shown in Table 3, it was confirmed that the parent strain *Corynebacterium glutamicum* CA09-0903 produced about 3.4 g/L of threonine. Among the strains expressing *pntAB* derived from foreign microorganisms, the strain expressing *pntAB* derived from *Edwardsiella tarda*, CA09-0903 △N1287(13032)::PgapA_pntAB(E.ta), exhibited a threonine-producing ability increased by 74% relative to the parent strain.

### Example 2-2: Construction of L-arginine-producing strains into which heterologous pntAB was introduced and evaluation of arginine-producing ability

Among the five types of vectors constructed in Example 1, vectors expressing *pntAB* derived from *Edwardsiella tarda*, which showed superior improvement in the producing ability in Example 2-1, were introduced into an arginine-producing strain. Specifically, transformation was performed by electroporation into an arginine-producing strain, *Corynebacterium glutamicum* CJ1R (Korean Patent Application No. 10-2021-0045262), to construct strains expressing heterologous *pntAB*.

In order to examine arginine productivity of the strains, culturing was performed as follows.

First, each strain was inoculated into a 250-mL corner-baffled flask containing 25 mL of seed medium and incubated with shaking at 200 rpm at 30°C for 20 hours. Then, 1 mL of the seed culture was inoculated into a 250-mL corner-baffled flask containing 24 mL of production medium and incubated with shaking at 200 rpm at 30°C for 72 hours.

### [Seed Medium (pH 7.0)]

Glucose 20 g, Peptone 10 g, Yeast Extract 5 g, Urea 1.5 g, KH₂PO₄ 4 g, K₂HPO₄ 8 g, MgSO₄·7H₂O 0.5 g, Biotin 100 µg, Thiamine HCl 1000 µg, Calcium Pantothenate 2000 µg, and Nicotinamide 2000 µg (per 1 L of distilled water).

### [Production Medium (pH 7.0)]

Glucose 6%, Ammonium Sulfate 3%, Potassium Dihydrogen Phosphate 0.1%, Magnesium Sulfate Heptahydrate 0.2%, Corn Steep Liquor (CSL) 1.5%, NaCl 1%, Yeast Extract 0.5%, and Biotin 100 mg/L

After completion of culturing, the amount of L-arginine produced was measured using high-performance liquid chromatography (HPLC), and the concentrations are shown in Table 4 below.

**[Table 4]**

| Arginine-producing ability of microorganisms expressing heterologous *pntAB* | | |
|---|---|---|
| Strain Name | L-arginine concentration (g/L) | Concentration relative to parent strain (%) |
| CJ1R (parent strain) | 2.0 | 100 |
| CJ1R △N1287(13869)::PgapA_pntAB (E.ta) | 2.72 | 136 |

As a result, as shown in Table 4, it was confirmed that the parent strain *Corynebacterium glutamicum* CJ1R produced about 2.0 g/L of arginine. The strain expressing *pntAB* derived from *Edwardsiella tarda*, CJ1R △N1287(13869)::PgapA_pntAB (E.ta), exhibited an arginine-producing ability increased by 36% relative to the parent strain.

### Example 2-3: Construction of L-homoserine-producing strains into which heterologous pntAB was introduced and evaluation of L-homoserine-producing ability

Among the five types of vectors constructed in Example 1, the vector expressing *pntAB* derived from *Edwardsiella tarda*, which showed superior improvement in the producing ability in Example 2-1, was transformed by electroporation into a homoserine-producing strain, *Corynebacterium glutamicum* KCCM 12120P (Korean Patent No. 10-1947959), to construct strains expressing heterologous *pntAB*.

In order to examine homoserine productivity of the strains, culturing was performed as follows.

Specifically, each strain was inoculated into a 250-mL corner-baffled flask containing 25 mL of production medium and incubated with shaking at 200 rpm at 30°C for 24 hours. After completion of culturing, the amount of L-homoserine produced was measured using HPLC, and the concentrations are shown in Table 5 below.

### [Production Medium (pH 7.0)]

Glucose 45 g, (NH₄)₂SO₄ 20 g, MgSO₄·7H₂O 1.2 g, KH₂PO₄ 1.1 g, Biotin 900 µg, Thiamine HCl 4500 µg, Calcium Pantothenate 4500 µg, and CaCO₃ 30 g (per 1 L of distilled water).

After completion of culturing, the amount of L-homoserine produced was measured using high-performance liquid chromatography (HPLC), and the concentrations are shown in Table 5 below.

**[Table 5]**

| Homoserine-producing ability of microorganisms expressing heterologous *pntAB* | | |
|---|---|---|
| Strain Name | L-homoserine concentration (g/L) | Concentration relative to parent strain (%) |
| KCCM12120P (parent strain) | 0.40 | 100 |
| KCCM12120P | 0.64 | 160 |
| △N1287(13032)::PgapA_pntAB(E.ta) | | |

As a result, as shown in Table 5, it was confirmed that the parent strain *Corynebacterium glutamicum* KCCM12120P produced about 0.4 g/L of homoserine. Among the strains expressing *pntAB* derived from foreign microorganisms, the strain expressing *pntAB* derived from *Edwardsiella tarda*, KCCM12120P △N1287(13032)::PgapA_pntAB(E.ta), exhibited a homoserine-producing ability increased by 60% relative to the parent strain.

### Example 2-4: Construction of L-valine-producing strains into which heterologous pntAB was introduced and evaluation of L-valine-producing ability

Among the five types of vectors constructed in Example 1, vectors expressing *pntAB* derived from *Edwardsiella tarda*, which showed superior improvement in the producing ability in Example 2-1, were introduced into a valine-producing strain. Specifically, transformation was performed by electroporation into a valine-producing strain, *Corynebacterium glutamicum* KCCM 11201P (U.S. Patent No. 8,465,962 B), to construct a strain expressing heterologous *pntAB*.

In order to examine valine productivity of the strains, culturing was performed as follows.

First, each strain was inoculated into a 250-mL corner-baffled flask containing 25 mL of seed medium and incubated with shaking at 200 rpm at 30°C for 20 hours. Then, 1 mL of the seed culture was inoculated into a 250-mL corner-baffled flask containing 24 mL of production medium and incubated with shaking at 200 rpm at 30°C for 72 hours.

### [Seed Medium (pH 7.0)]

Glucose 20 g, Peptone 10 g, Yeast Extract 5 g, Urea 1.5 g, KH₂PO₄ 4 g, K₂HPO₄ 8 g, MgSO₄·7H₂O 0.5 g, Biotin 100 µg, Thiamine HCl 1000 µg, Calcium Pantothenate 2000 µg, and Nicotinamide 2000 µg (per 1 L of distilled water).

### [Production Medium (pH 7.0)]

Glucose 6%, Ammonium Sulfate 3%, Potassium Dihydrogen Phosphate 0.1%, Magnesium Sulfate Heptahydrate 0.2%, Corn Steep Liquor (CSL) 1.5%, NaCl 1%, Yeast Extract 0.5%, and Biotin 100 mg/L

After completion of culturing, the amount of L-valine produced was measured using high-performance liquid chromatography (HPLC), and the concentrations are shown in Table 6 below.

**[Table 6]**

| Valine-producing ability of microorganisms expressing heterologous *pntAB* | | |
|---|---|---|
| Strain Name | L-valine concentration (g/L) | Concentration relative to parent strain (%) |
| KCCM11201P | 2.7 | 100 |
| KCCM11201P | 2.9 | 107 |
| △N1287(14067)::PgapA_pntAB(E.ta) | | |

As a result, as shown in Table 6, it was confirmed that the parent strain *Corynebacterium glutamicum* KCCM11201P produced about 2.7 g/L of valine. The strain expressing *pntAB* derived from *Edwardsiella tarda*, KCCM11201P △N1287(14067)::PgapA_pntAB(E.ta), exhibited a valine-producing ability increased by 7.4% relative to the parent strain.

### Example 2-5: Construction of L-lysine-producing strains into which heterologous pntAB was introduced and evaluation of L-lysine-producing ability

Among the five types of vectors constructed in Example 1, vectors expressing *pntAB* derived from *Edwardsiella tarda*, which showed superior improvement in the producing ability in Example 2-1, were introduced into a lysine-producing strain. Specifically, transformation was performed by an electric pulse method (Van der Rest et al., Appl. Microbiol. Biotechnol. 52:541-545, 1999) into a lysine-producing strain, *Corynebacterium glutamicum* CJ3P (U.S. Patent No. 9,556,463 B2), to construct a strain expressing heterologous *pntAB*.

In order to examine lysine productivity of the strains, culturing was performed as follows.

First, each strain was inoculated into a 250 mL corner-baffled flask containing 25 mL of seed medium, and incubated with shaking at 37°C for 20 hours at 200 rpm. Thereafter, 1 mL of the seed culture was inoculated into a 250 mL corner-baffled flask containing 24 mL of production medium, and incubated with shaking at 37°C for 36 hours at 200 rpm.

### [Seed Medium (pH 7.0)]

Glucose 20 g, Peptone 10 g, Yeast Extract 5 g, Urea 1.5 g, KH₂PO₄ 4 g, K₂HPO₄ 8 g, MgSO₄·7H₂O 0.5 g, Biotin 0.1 mg, Thiamine HCl 1 mg, Calcium Pantothenate 2 mg, and Nicotinamide 2 mg (per 1 L of distilled water).

### [Production Medium (pH 7.0)]

Glucose 100 g, (NH₄)₂SO₄ 40 g, Soybean Protein 2.5 g, Corn Steep Solids 5 g, Yeast 3 g, KH₂PO₄ 1 g, MgSO₄·7H₂O 0.5 g, Biotin 100 µg, Thiamine HCl 1000 µg, Calcium Pantothenate 2000 µg, Nicotinamide 3000 µg, and CaCO₃ 30 g (per 1 L of distilled water).

After completion of culturing, the amount of L-lysine produced was measured using high-performance liquid chromatography (HPLC), and the concentrations are shown in Table 7 below.

**[Table 7]**

| Lysine-producing ability of microorganisms expressing heterologous *pntAB* | | |
|---|---|---|
| Strain Name | L-lysine | Concentration relative |
| | concentration (g/L) | to parent strain (%) |
| CJ3P | 7.51 | 100 |
| CJ3P△N1287(13032)::PgapA_pntAB (E.ta) | 7.9 | 105.3 |

As a result, as shown in Table 7, it was confirmed that the parent strain *Corynebacterium glutamicum* CJ3P produced about 7.51 g/L of lysine. The strain expressing *pntAB* derived from *Edwardsiella tarda*, CJ3P△N1287(13032)::PgapA_pntAB (E.ta), exhibited a lysine-producing ability increased by 5.3% relative to the parent strain.

The above results confirmed that, among the five enzymes selected in the present disclosure, introduction of the gene encoding *pntAB* derived from *Edwardsiella tarda* increased reducing power through *pntAB* activity, thereby enabling more efficient production of L-amino acids and derivatives thereof.

As set forth above, those skilled in the art will be able to understand that the present disclosure may be embodied in other specific forms without departing from the technical spirit or essential characteristics thereof. In this regard, it should be understood that the foregoing examples are illustrative in all respects and are not to be construed as limiting. The scope of the present disclosure should be construed as including the meaning and scope of the appended claims rather than the detailed description, and all changes or variations derived from the equivalent concepts fall within the scope of the present disclosure.

## Claims

1. A microorganism of the genus *Corynebacterium* having improved ability to produce an L-amino acid or a derivative thereof, into which a polynucleotide encoding a nicotinamide nucleotide transhydrogenase protein derived from *Edwardsiella tarda* is introduced.

2. The microorganism according to claim 1, wherein the nicotinamide nucleotide transhydrogenase derived from *Edwardsiella tarda* is encoded by a *pntAB* gene.

3. The microorganism according to claim 1, wherein the nicotinamide nucleotide transhydrogenase derived from *Edwardsiella tarda* consists of an alpha subunit PntA and a beta subunit PntB.

4. The microorganism according to claim 1, wherein the alpha subunit PntA of the nicotinamide nucleotide transhydrogenase derived from *Edwardsiella tarda* comprises an amino acid sequence of SEQ ID NO: 18 or an amino acid sequence having at least 80% sequence identity thereto, and the beta subunit PntB comprises an amino acid sequence of SEQ ID NO: 19 or an amino acid sequence having at least 80% sequence identity thereto.

5. The microorganism according to claim 1, wherein the microorganism of the genus *Corynebacterium* having improved ability to produce an L-amino acid or a derivative thereof is *Corynebacterium glutamicum*.

6. The microorganism according to *a*ny one of claims 1 to 5, wherein the microorganism of the genus *Corynebacterium* has improved ability to produce an L-amino acid or a derivative thereof compared to a non-modified microorganism, wherein the L-amino acid is at least one selected from L-lysine, L-valine, L-arginine, L-threonine, and L-homoserine.

7. A method for producing an L-amino acid or a derivative thereof, comprising culturing, in a medium, a microorganism of the genus *Corynebacterium* having improved ability to produce an L-amino acid or a derivative thereof, into which a polynucleotide encoding a nicotinamide nucleotide transhydrogenase protein derived from *Edwardsiella tarda* is introduced.

8. The method according to claim 7, further comprising recovering the L-amino acid or a derivative thereof in the cultured microorganism, a culture of the microorganism, a fermentation product of the microorganism, or the culture medium.

9. The method according to claim 7, wherein the L-amino acid or a derivative thereof is at least one selected from L-lysine, L-valine, L-arginine, L-threonine, and L-homoserine.

10. A composition for producing an L-amino acid or a derivative thereof, comprising: a microorganism of the genus *Corynebacterium* having improved ability to produce an L-amino acid or a derivative thereof, into which a polynucleotide encoding a nicotinamide nucleotide transhydrogenase protein derived from *Edwardsiella tarda* is introduced; a culture of the microorganism; a fermentation product of the microorganism; or a combination of two or more thereof, .

11. Use of a microorganism of the genus *Corynebacterium* having improved ability to produce an L-amino acid or a derivative thereof, into which a nicotinamide nucleotide transhydrogenase protein derived from *Edwardsiella tarda* or a polynucleotide encoding the same is introduced, for producing the L-amino acid or a derivative thereof.
